# EUROPEAN PATENT SPECIFICATION

(11) **EP 4 372 205 B1**
(45) Date of publication and mention of the grant of the patent: **17.12.2025**
(21) Application number: 22765929.9
(22) Date of filing: 13.07.2022
(51) Int. Cl.: E21B 49/08, G01N 33/18

(54) **AQUIFER CONTAMINATION MONITORING PROCEDURE AND COLLECTION DEVICE FOR THIS PURPOSE**
GRUNDWASSERLEITERVERSCHMUTZUNGSÜBERWACHUNGSVERFAHREN UND SAMMELVORRICHTUNG DAFÜR
PROCÉDÉ DE SURVEILLANCE DE CONTAMINATION D'AQUIFÈRES ET DISPOSITIF DE CAPTAGE À CET EFFET

(30) Priority: 14.07.2021 ES 202130664
(43) Date of publication of application: 22.05.2024
(73) Proprietor: Beltrán Medina, Pedro, 46018 Valencia (ES); Martínez Arias, Alfredo, 30100 Murcia (ES)
(72) Inventor: Beltrán Medina, Pedro, 46018 Valencia (ES); Martínez Arias, Alfredo, 30100 Murcia (ES)
(74) Representative: Galbaian S.Coop.
(86) International application number: PCT/ES2022/070455
(87) International publication number: WO 2023/285724

(56) References cited:
- US-A1- 2009 038 390
- US-A1- 2020 072 737

## Description

### TECHNICAL FIELD

The present invention relates to a collection device and a procedure for monitoring the leaching of contaminants into the aquifer, in particular for the presence of compounds such as nitrates from leaching of fertilised soils or nitrogenous discharges.

It is applicable in the fields of environment, agriculture, livestock, industry and hydrogeology.

### PRIOR ART

A relevant environmental problem is the depletion and contamination of underground and surface water resources. The overexploitation of aquifers and the contamination by materials from the surface render this resource unusable, whose renewal is multiannual.

For example, one known problem is contamination by nitrates and similar compounds used in agriculture or produced in livestock farming. In order to reduce this problem, a series of standards and control measures have been defined to reduce the risks of contamination of aquifers and contamination, eutrophication of associated surface water bodies.

However, it is necessary to control to what extent these measures are effective, for which systems such as the one disclosed in US6021664 have been developed for monitoring aquifer quality.

The method of US6021664 is a method for collecting and/or determining water in the saturated medium. The saturated medium contains a mixture of all the water in the aquifer, therefore, it is not possible to detect the origin of any contamination. It is necessary to identify and determine whether the leachate water from a particular point contributes contamination.

Furthermore, current measurements detect the presence of compounds in the saturated zone of the aquifer, without it being easy to locate their origin. It is therefore preferable to collect them before they reach the aquifer, in the unsaturated zone, already in an undisturbed area out of the reach of agricultural work (disturbed area), but above the water table, in order to collect the water that flows through the disturbed area and carries the leached compounds with it.

GB2587587A is also cited as an example of a system that performs underground measurements, isolating the area to prevent contamination.

The applicant is unaware of any collection procedure or device that could be considered similar to the invention.

US2009038390A1 shows a method and system for monitoring soil properties and for collecting soil pore-water samples.

### DISCLOSURE OF THE INVENTION

The invention relates to an aquifer contamination monitoring procedure and a collection device for this purpose, according to the independent claims. Their various examples solve the problems of the prior art and provide significant advantages.

The collection device comprises a leachate collection system for real-time chemical characterisation, located in the unsaturated zone of the soil, below the disturbed area of arable soil. The collection device has three main objectives:
- Confirm whether leachate or drainage caused by washing occurs below the disturbed area of the arable soil in question.
- Characterise in real time the concentration of contaminating compounds in the leachate, e.g. nitrates, chlorides, arsenic, heavy metals, potassium or phosphates.
- Avoid altering the physicochemical conditions of the soil, avoiding the suction of liquids or withdrawal of liquids. The liquid enters by gravity and, in some examples, also exits by gravity.

For this, an aquifer monitoring procedure is defined using sensors that record the concentrations of chemical elements in the leachate water coming from the surface, using a collection chamber of said water. It starts with a first stage of construction of a collection device through the phases of:
a. Determine the depth of the disturbed area by workings, to be able to place the probe below that area. The water table is also relevant, but it is often well below the disturbed area, so it is not always necessary to consider it.
b. Determine the permeability of the soil below the disturbed area.
c. Define an inclination, with respect to the horizontal, of a borehole as a function of the determined permeability.
d. If applicable, define a section of a discharge orifice of the chamber based on the determined permeability, to retain the water long enough for its determination.
e. Perform the borehole with the defined inclination and introduce the collection chamber with an upper collection seepage pipe and the defined discharge orifice at the bottom of the collection chamber.

Once this collection device, which is part of the invention, has been built, a second stage is carried out to determine the parameters being monitored and/or to send them to an external point. If the collection chamber is watertight (without a discharge or drainage orifice), the sample can be taken by pumping and the determinations can be made "in situ", or by sending the sample to the laboratory. If the measurements are made by probes, in real time, the collection chamber has sensors to analyse the water as it escapes or is renewed through the discharge orifice. The sensors can measure physicochemical parameters (conductivity, pH, dissolved oxygen, redox, alkalinity, etc) or chemical parameters (e.g. by absorption spectrometers).

Other variants will be shown in the remainder of the description.

### DESCRIPTION OF THE DRAWINGS

For a better understanding of the invention, the following figures are included.
Figure 1: General view of an example.
Figure 2: Schematic detail of the previous example.

### DETAILED DISCLOSURE OF THE INVENTION

Next, an embodiment of the invention is briefly described as an illustrative and non-limiting example.

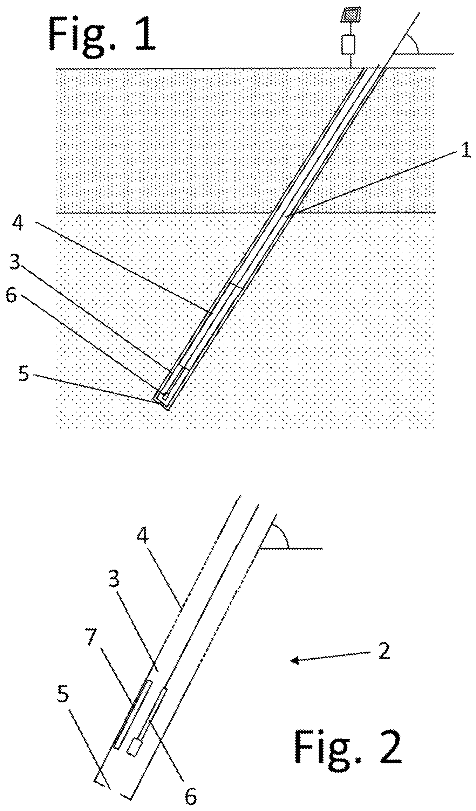

Figure 1 shows an example formed by a conduit (1) that forms a well with a collector (2) comprised of a collection chamber (3) or container with a perforated upper seepage pipe (4) with a perimeter filter and a lower discharge orifice (5). Generally the collector is located in the lowest part of the well, but it can also be placed at an intermediate point depending on the situation of the water table. The collector (2) is isolated from the other layers by a watertight seal, as known in the art; for example, using cementation with bentonite in the ring around the conduit (1) up to the surface, and cement grout could be used in the upper section (for example, 20 cm). The seepage pipe (4), with an opening for light depending on the granulometry of the geological formation, is surrounded by a homometric siliceous gravel packing or some other inert material. The length of the seepage pipe (4) mainly depends on the situation of the water table.

The seal ensures that the leachate introduced into the chamber (3) is of a specific depth or stratum and that there are no contributions of water from higher levels of the disturbed area of the soil. This way, the leachate that passes through the collection chamber corresponds to the leachate that passes into the undisturbed area of the soil without mixing with leachate from different origins.

The interior of the chamber (3) comprises a physicochemical or chemical sensor (6) which is preferably an absorption spectrometer that takes the measurements in real-time (continuously or with a specific programmable frequency). For example, attenuation will be monitored around a wavelength of 212 nm to check for the presence of nitrates. Thanks to the additional measurement of the attenuation at the 254 nm and 360 nm wavelengths, interferences from organic matter and water turbidity can be compensated using the principle of cross-sensitivity. This type of spectrometer is commercially available.

A level (7) can also be placed inside the chamber (3) to perform the measurements when there is sufficient liquid inside, when the chamber (3) is filled by lowering water from the surface. That level (7) can also send readings to the surface. The level (7) can be binary (sufficient height has been reached - not reached) and block or authorise readings or pumping.

The data from the sensors (6,7) are collected and transmitted to the surface, from where they are sent to the control centre. The wiring can be carried by centralisers, as known in the art of drilling.

In a novel way, the chamber (3) with its perforated pipe (4) and orifice (5) are arranged depending on the permeability of the layer where it is installed. To do this, the first step in the monitoring procedure is to characterise the layer where the chamber will be installed to assess its permeability. The inclination (*pitch*) of the chamber (3) and the section where the orifice (5) is located are related to this permeability. This way, sufficient residence time of the liquid inside the chamber (3) and the sufficient level reached for the readings to be correct can be ensured. When the permeability is high, the orifice (5) is small since it must retain the liquid long enough for its determination. If the permeability is low, the orifice (5) will be larger to prevent the residence time from increasing in the chamber and ensure its renewal.

The following table sets out the conditions according to permeability, in the case of an orifice (5) with a circular cross-section. Other shapes for the orifice (5) shall have an equivalent cross-section to ensure that the measured water outlet occurs at the same or a similar speed. The load losses due to the shape of the orifice (5) are reduced; therefore, they can be neglected.

| Permeability | Inclination of the chamber | Diameter |
|---|---|---|
| < 10⁻⁸ m/s | 30-40° | 20 mm |
| 10⁻⁸ - 10⁻⁶ m/s | 40-50° | 15 mm |
| 10⁻⁶ - 10⁻⁴ m/s | 50-60° | 10 mm |
| 10⁻⁴ - 10⁻² m/s | 60-70° | 5 mm |
| > 10⁻² m/s | 70-80° | 2 mm |

The depth and degree of inclination of the collection chamber (3) shall be fixed "a priori" according to the previously performed vulnerability study: type of plant and surface fertilizer plan, rainfall or irrigation, power of the disturbed area...and permeability measured in other boreholes (or in this one). Permeability tests will be carried out during drilling at the different levels detected in the cores. The seepage pipe and the collection chamber will be placed in the best position for the collection and determination of the leachate. Generally, drilling will be from 3 to 10 meters, depending on the area.

In one example, in areas of low vulnerability, the collection chamber (3) shall be sealed (without orifice) and the sample shall be pumped out and either determined in situ or sent to the laboratory.

An important point of the invention is to detect the depth or thickness of the disturbed area and the permeability of the unsaturated zone in the undisturbed area. A valid method is to perform a borehole with continuous core taking for examination by a geologist, carrying out "Lefranc" permeability tests at each permeable level detected. The drilling in this case will be vertical for simplicity. The disturbed area is generally 0.5-1.5 metres thick, but a borehole of this type will almost always be greater than 3 m and the total length until the saturated zone is reached. In this way, all the levels will be hydrodynamically defined until the saturated zone, which is the object of protection, is reached.

Thus, the total depth of the device will depend on the thickness of the disturbed area and the permeability of the undisturbed area, where samples must be taken, since the seepage pipe must be placed from that depth on, generally with a certain margin of safety. Likewise, the length of the seepage pipe can vary according to permeability.

## Claims

1. Aquifer contamination monitoring procedure, by means of sensors (6,7), of the leaching water circulating in the unsaturated zone of the soil coming from the surface, by determining chemical or physico-chemical parameters of the water present in a collection chamber (3) **characterized by**
comprising a first stage of construction of a collection device, by carrying out the following phases:
determine the depth of a disturbed area of the soil disturbed by agricultural work, wherein the unsaturated zone is located below said disturbed area;
determine the permeability of the soil below the disturbed area;
define an inclination of a borehole according to the determined permeability;
carry out the borehole with the defined inclination and introduce the collection chamber (3) with an upper collection seepage pipe (4); and
a second stage of parameter determination and submission of readings.

2. Aquifer contamination monitoring procedure, according to claim 1, **characterised in that** the upper collection seepage pipe (4) has a perimeter filter and is surrounded by a homometric siliceous gravel packing or some other inert material.

3. Aquifer contamination monitoring procedure, according to claim 1 or 2, **characterised in that** the sensors (6,7) include a chemical sensor (6).

4. Aquifer contamination monitoring procedure, according to claim 3, **characterised in that** the chemical sensor (6) is an absorption spectrometer.

5. Aquifer contamination monitoring procedure, according to any preceding claim, **characterised in that** it comprises a step of defining a section of a discharge orifice (5) of the collection chamber (3) based on the determined permeability; creating a discharge orifice (5) at the bottom of the collection chamber (3) prior to its introduction into the borehole; and place one or more sensors (6,7) in the collection chamber (3) for perform the determinations.

## Patentansprüche

1. Verfahren zur Überwachung der Grundwasserleiterverschmutzung mittels Sensoren (6, 7) des in der ungesättigten Zone des Bodens zirkulierenden, von der Oberfläche kommenden Sickerwassers durch Bestimmen chemischer oder physikalisch-chemischer Parameter des in einer Sammelkammer (3) vorhandenen Wassers, **dadurch gekennzeichnet, dass** es eine erste Stufe der Konstruktion einer Sammelvorrichtung, in der die folgenden Phasen durchgeführt werden:
Bestimmen der Tiefe eines gestörten Bereichs des durch landwirtschaftliche Arbeiten gestörten Bodens, wobei sich die ungesättigte Zone unterhalb des gestörten Bereichs befindet;
Bestimmen der Durchlässigkeit des Bodens unterhalb des gestörten Bereichs;
Definieren einer Neigung eines Bohrlochs gemäß der bestimmten Durchlässigkeit;
Ausführen des Bohrlochs mit der definierten Neigung und Einführen der Sammelkammer (3) mit einem oberen Sammelsickerrohr (4); und
eine zweite Stufe der Parameterbestimmung und Übermittlung der Messwerte umfasst.

2. Verfahren zur Überwachung der Grundwasserleiterverschmutzung nach Anspruch 1, **dadurch gekennzeichnet, dass** das obere Sammelsickerrohr (4) einen Umfangsfilter aufweist und von einer homometrischen Silikatkiespackung oder einem anderen inerten Material umgeben ist.

3. Verfahren zur Überwachung der Grundwasserleiterverschmutzung nach Anspruch 1 oder 2, **dadurch gekennzeichnet, dass** die Sensoren (6, 7) einen chemischen Sensor (6) enthalten.

4. Verfahren zur Überwachung der Grundwasserleiterverschmutzung nach Anspruch 3, **dadurch gekennzeichnet, dass** der chemische Sensor (6) ein Absorptionsspektrometer ist.

5. Verfahren zur Überwachung der Grundwasserleiterverschmutzung nach einem der vorhergehenden Ansprüche, **dadurch gekennzeichnet, dass** es einen Schritt des Definierens eines Abschnitts einer Auslassöffnung (5) der Sammelkammer (3) basierend auf der bestimmten Durchlässigkeit; Erzeugen einer Auslassöffnung (5) am Boden der Sammelkammer (3) vor ihrer Einführung in das Bohrloch; und Platzieren eines oder mehrerer Sensoren (6, 7) in der Sammelkammer (3) zum Durchführen der Bestimmungen umfasst.

## Revendications

1. Procédé de surveillance de la contamination d'un aquifère, au moyen de capteurs (6,7), de l'eau de lixiviation circulant dans la zone non saturée du sol provenant de la surface, par détermination de paramètres chimiques ou physico-chimiques de l'eau présente dans une chambre de collecte (3), **caractérisé en ce qu'**il comprend une première étape de construction d'un dispositif de collecte en réalisant les phases suivantes:
déterminer la profondeur d'une zone perturbée du sol perturbée par des travaux agricoles, dans laquelle la zone non saturée est située en dessous de ladite zone perturbée;
déterminer la perméabilité du sol en dessous de la zone perturbée;
définir une inclinaison d'un forage en fonction de la perméabilité déterminée;
réaliser le forage avec l'inclinaison définie et introduire la chambre de collecte (3) avec un tuyau supérieur de collecte par infiltration (4); et
une seconde étape de détermination de paramètres et de soumission de lectures.

2. Procédé de surveillance de la contamination d'un aquifère, selon la revendication 1, **caractérisé en ce que** le tuyau supérieur de collecte par infiltration (4) présente un filtre périphérique et est entouré d'un remblai de gravier siliceux homométrique ou d'un autre matériau inerte.

3. Procédé de surveillance de la contamination d'un aquifère, selon la revendication 1 ou 2, **caractérisé en ce que** les capteurs (6,7) comprennent un capteur chimique (6).

4. Procédé de surveillance de la contamination d'un aquifère, selon la revendication 3, **caractérisé en ce que** le capteur chimique (6) est un spectromètre d'absorption.

5. Procédé de surveillance de la contamination d'un aquifère, selon l'une quelconque des revendications précédentes, **caractérisé en ce qu'**il comprend une étape de définition d'une section d'un orifice d'évacuation (5) de la chambre de collecte (3) sur la base de la perméabilité déterminée; la création d'un orifice d'évacuation (5) au fond de la chambre de collecte (3) avant son introduction dans le forage; et le placement d'un ou plusieurs capteurs (6,7) dans la chambre de collecte (3) pour effectuer les déterminations.
